(19) Europäisches Patentamt
European Patent Office
Office européen des brevets

(11) **EP 4 257 033 A1**

(12) **EUROPEAN PATENT APPLICATION**

(43) Date of publication:
**11.10.2023 Bulletin 2023/41**

(21) Application number: **22166556.5**

(22) Date of filing: **04.04.2022**

(51) International Patent Classification (IPC):
*A61B 5/00* *(2006.01)*

(52) Cooperative Patent Classification (CPC):
**A61B 5/725; A61B 5/7203**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **KAHLMAN, Josephus Arnoldus Johannes Maria**
  Eindhoven (NL)
• **GROSFELD, Ronny Hubertus Johannes**
  Eindhoven (NL)
• **VERSTRAELEN, Martinus Johannes Wilhelmina**
  Eindhoven (NL)

(74) Representative: **Philips Intellectual Property & Standards**
**High Tech Campus 52**
**5656 AG Eindhoven (NL)**

(54) **FILTERING A CARDIAC SIGNAL**

(57) A mechanism for filtering power-line interference noise out of a cardiac signal. One or more linear filters, including at least a feedback comb filter, are used to produce a first version of the power-line interference signal. This is then subtracted from the cardiac signal to produce the filtered cardiac signal.

FIG. 1

**Description**

TECHNICAL FIELD

The present disclosure relates to the field of cardiac signals, and in particular, to the post-processing of cardiac signals.

BACKGROUND

An increasingly important method for monitoring the condition of a subject's heart is the use of cardiac signals, which represent an electrical activity of the subject's heart. A wide variety of cardiac signals are used in the healthcare profession to assess or monitor the heart, including electrocardiogram (ECG) signals and/or internal heart signals.

An ongoing problem with cardiac signals is the presence of noise, particularly a power-line interference (PLI) signal. The PLI signal can be characterized as a periodic signal with a fundamental frequency that matches the power-line frequency (around 50 Hz or 60Hz), plus multiple harmonics of various amplitudes and phases. Disadvantageously, the clinically important frequency band of many cardiac signals (such as the ECG signal) spans a range from a DC frequency (0Hz) to approximately 1 kHz. This implies that both the fundamental PLI frequency and its harmonics fall into mentioned frequency range, causing substantial interference and noise to cardiac signals, significantly impacting upon a clinical usefulness of cardiac signals.

There is therefore a need to reduce noise in cardiac signals, particularly noise attributable to PLI signals. A typical approach for reducing such noise is to use a cascade or array of narrow band-rejection filters, which are tuned to the relevant PLI harmonics.

Improved and more flexible approaches are desired.

SUMMARY OF THE INVENTION

The invention is defined by the claims.

According to examples in accordance with an aspect of the invention, there is provided a processing system for filtering a cardiac signal representative of electrical activity of a portion of a subject's heart. The processing system is configured to: obtain, at an input interface, the cardiac signal; process the cardiac signal using only a first set of one or more linear filters, including at least a feedback comb filter, to extract or isolate a first version of a power-line interference signal in the cardiac signal, subtract the first version of the power-line interference signal from the cardiac signal to produce a filtered cardiac signal; and optionally, output, at an output interface, the filtered cardiac signal.

Embodiments provide a technique for filtering power-line interference noise from a cardiac signal. In particular, embodiments propose performing only linear filtering on the cardiac signal, which includes the use of a feedback comb filter. A feedback comb filter provides a reliable and accurate mechanism for filtering noise that occurs at a sequence of evenly spaced apart frequencies. It is recognized that power-line interference occurs at a fundamental frequency and the harmonics thereof, such that a feedback comb filter provides a particularly suitable mechanism for extracting a power-line interference signal.

The use of only one or more linear filters preserves noise characteristics of the power-line interference noise. This means that the extracted power-line interference signal more closely resembles the true power-line interference noise (e.g., compared to a synthesized version of the PLI noise).

A version of a power-line interference signal is a signal that represents a prediction or determination of the power-line interference signal in or carried by the cardiac signal. The term "version of a power-line interference signal" could therefore be replaced by the term "predicted power-line interference signal".

In some examples, the processing system is further configured to: process the cardiac signal to detect changes in the peak amplitude and/or phase of the power-line interference signal in the cardiac signal; and responsive to a detected change in the peak amplitude and/or phase, modify the scaling factor for the feedback comb filter to reduce a settle time of the feedback comb filter.

This approach provides a mechanism for controlling the feedback comb filter to quickly respond to changes in the PLI noise, reducing a time at which the filtered cardiac signal inaccurately represents the ground-truth cardiac signal. By modifying the settle time in this way, a more accurate representation of the ground-truth cardiac signal can be achieved. Put another way, the filter is able to quickly adapt to mentioned changes in amplitude(s) and/or phase(s) of the PLI harmonic(s), and is therefore able to consistently and accurately output the representative cardiac signal.

The reduction to the settle time may be temporary, e.g., so that the feedback comb filter can perform a low accuracy filtering approach to quickly adapt to the change in PLI conditions before reverting to a more accurate filtering approach.

In some examples, the processing system is configured to detect changes in the peak amplitude and/or phase of the power-line interference signal in the cardiac signal by: processing the cardiac signal using only a second set of one or more linear filters, including at least a second feedback comb filter, to extract or isolate a second version of the power-line interference signal carried by the cardiac signal, wherein a settle time of the second feedback comb filter is less than the settle time of the feedback comb filter; monitoring a difference between the first version of the power-line interference signal and the second version of the power-line interference signal; and responsive to a difference exceeding a first predetermined difference threshold, determining that there is change in the peak amplitude and/or phase of the power-line interference signal in the cardiac signal.

This approach provides a reliable and fast response mechanism for detecting any changes in the power-line interference carried by the cardiac signal. Thus, a low-latency approach for responding to changes in power-line interference is achieved.

In some examples, the processing system is further configured to, responsive to the monitored difference between the first version of the power-line interference signal and the second version of the power-line interference signal falling below a second predetermined difference threshold, modify the scaling factor for the feedback comb filter to increase the settle time of the feedback comb filter. The first and second predetermined thresholds may be the same or different.

The processing system may be configured to modify the scaling factor for the feedback comb filter to decrease the settle time of the feedback comb filter by changing the scaling factor from a first value to a second, different value.

Similarly, the processing system may be configured to modify the scaling factor for the feedback comb filter to increase the settle time of the feedback comb filter by changing the scaling factor from the second value to the first value.

In this way, if the scaling factor of the feedback comb filter had been modified by the processing system responsive to the difference exceeding a first predetermined difference threshold, increasing the scaling factor may comprise controlling the scaling factor to be equal to the scaling factor before the modification responsive to the difference exceeding a first predetermined difference threshold.

The feedback comb filter may be configured to generate the first version of the power-line interference signal by: summing the cardiac signal with a first feedback signal to produce the first version of the power-line interference signal; delaying the first version of the power-line interference signal by a delay length to produce a delayed PLI signal; subtracting the cardiac signal from the delayed PLI signal to produce a second feedback signal; and multiplying the second feedback signal by a scaling factor to produce the first feedback signal.

In some examples, the processing system is further configured to process the cardiac signal to identify a fundamental frequency of a power-line interference signal in the cardiac signal, wherein a delay length for the feedback comb filter is responsive to the identified fundamental frequency of the power-line interference signal.

The processing system may be configured to process the cardiac signal to identify a resonant frequency of the power-line interference signal in the cardiac signal by performing a discrete Fourier-based transform on the cardiac signal.

The discrete Fourier-based transform may comprise a fast Fourier transform or a short-time fast Fourier transform. This provides a reliable and accurate approach for identifying a resonant frequency of the power-line interference signal. The amplitude at the frequency of the power-line interference signal will usually be significantly dominant over the amplitude(s) at other frequencies of the cardiac signal, particularly in a specific range of between 45Hz and 65Hz, where PLI is expected to occur.

It will be appreciated that other approaches for identifying a resonant frequency could be adopted by the appropriately skilled person.

The cardiac signal may be a high right atrial electrogram signal, a his-bundle electrogram signal or an electrocardiogram signal. These are examples of cardiac signals that are particularly susceptible to power-line interference.

The cardiac signal may be produced by an intravenous monitoring device such as a catheter. Due at least to the distance between an intravenous monitoring device and the external monitoring system, there is a significant increase in the effect of power-line interference on such signals, meaning that the proposed approach is particularly advantageous in such scenarios.

The processing system may be further configured to store the filtered cardiac signal in a memory or recording system. In some examples, the processing system may be further configured to control a user interface to provide a visual representation of the filtered cardiac system. In some examples, the filtered cardiac signal may be provided to a further processing system, e.g., a system configured to analyze or assess the condition of the subject based on the filtered cardiac signal and optionally other parameters or properties of the subject of the cardiac signal.

The processing system may be a field-programmable gate array or an application-specific integrated circuit. The use of linear filters alone in generating the first version of the power-line interference signal facilitates ease of implementation of an FPGA or ASIC. Another alternative processing system that could be used is a programmable processor.

There is also proposed a computer-implemented method for filtering a cardiac signal representative of electrical activity of a portion of a subject's heart. The computer-implemented method comprises: obtaining the cardiac signal; processing the cardiac signal using only a first set of one or more linear filters, including at least a feedback comb filter, to extract or isolate a first version of a power-line interference signal in the cardiac signal; subtracting the first version of the power-line interference signal from the cardiac signal to produce a filtered cardiac signal; and optionally, outputting the filtered cardiac signal.

In some examples, the (computer-implemented) method further comprises, processing the cardiac signal to detect changes in the peak amplitude and/or phase of the power-line interference signal in the cardiac signal; and responsive to a detected change in the peak amplitude and/or phase, modifying the scaling factor for the feedback comb filter to reduce a settle time of the feedback comb filter.

The skilled person would be readily capable of adapting the computer-implemented method to carry out the steps performed by any herein described processing system. Similarly, any herein described processing system may be appropriately configured for performing the steps of any herein described (computer-implemented) method.

There is also proposed a computer program product comprising computer program code means which, when executed on a computing device having a processing system, cause the processing system to perform all of the steps of any herein described method.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 illustrates an approach for filtering a cardiac signal;
Figure 2 illustrates an approach for extracting a power-line interference signal from a cardiac signal;
Figure 3 illustrates different filtered cardiac signals;
Figure 4 illustrates an approach for extracting a power-line interference signal from a cardiac signal;
Figure 5 illustrates the effect of dynamically modifying a settle time of a feedback comb filter used for extracting a power-line interference signal from a cardiac signal;
Figure 6 is a flowchart illustrating a method; and
Figure 7 illustrates an apparatus and a processing system.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for filtering power-line interference noise out of a cardiac signal. One or more linear filters, including at least a feedback comb filter, are used to produce a first version of the power-line interference signal. This is then subtracted from the cardiac signal to produce the filtered cardiac signal.

Embodiments are based on the realization that linear filtering alone, which includes using a feedback comb filter, can produce a reliable and accurate version of a power-line interface signal. Further embodiments are based on the realization that the coefficients or properties of the feedback comb filter can be defined or controlled to improve the performance and accuracy of extracting the power-line interference signal, and therefore of the cardiac signal.

Embodiments may be employed in any environment in which a cardiac signal is to be processed, but find particular use in medical environments such as those employing an intravenous catheter that monitor one or more cardiac signal within the subject.

The proposed system and approach provides a fast and responsive, with an extremely low latency, mechanism for filtering a cardiac signal that can be used to perform live or real-time filtering, and therefore is to particular advantage when employed in a clinical setting.

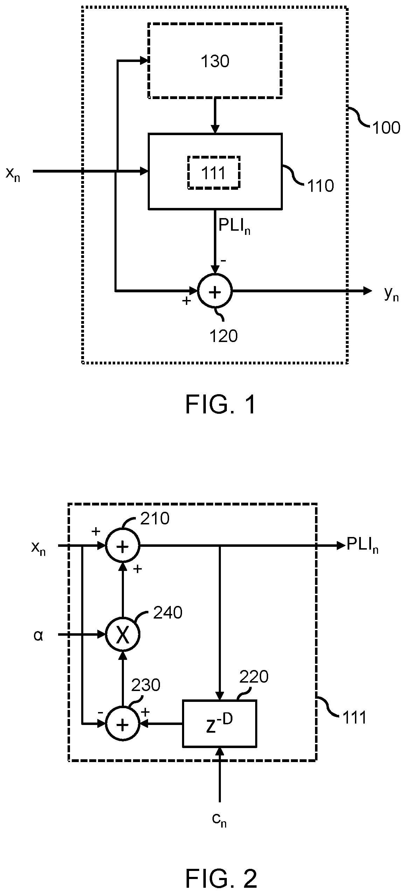

Figure 1 conceptually illustrates an approach for processing or filtering a cardiac signal $x_n$, which is here represented in a discrete-time form. Figure 1 may effectively represent a block diagram demonstrating the steps or procedures carried out by a processing system 100 for processing the cardiac signal $x_n$.

The cardiac signal $x_n$ may be a signal that was originally produced or generated by an electrode positioned to sense or monitor electrical activity of the heart. This electrode may be located on a catheter and/or externally to the subject. Thus, the cardiac signal may be produced by an intravenous monitoring device such as a catheter. As another example, the cardiac signal may be produced by an external electrode, such as used in an conventional lead-based electrocardiogram. The cardiac signal may be a recorded version of the signal obtained by the electrode or a signal received directly, "live" or in real-time from the electrode.

The cardiac signal $x_n$ may be a discretized or digital version of an analogue or continuous-time form signal x(t). In other examples, the cardiac signal $x_n$ is preserved in analogue form x(t).

Cardiac signals are considered to carry clinically relevant information in a frequency band of from 0.05Hz to 150Hz or so. Power-line interference is a recognized problem in affecting the interpretability of such cardiac signals. Typically, power-line interference has a fundamental component in a region of around 50-60Hz, although its harmonics also cause interference.

The cardiac signal may be a high right atrial electrogram signal, a his-bundle electrogram signal or an electrocardiogram signal. Other forms of suitable cardiac signal that suffer from, exhibit or carry a power-line interference signal would be readily apparent to the skilled person.

The proposed approach makes use of a first set 110 of one or more linear filters that filter the cardiac signal to produce a first version of the power-line interference signal $PLI_n$ in the cardiac signal $x_n$. A linear filter is a filter that processes an input signal subject to the constraint of linearity.

The first set 110 of one or more linear filters comprises at least a (first) feedback comb filter 111. In some examples, the first set 110 comprises only a feedback comb filter. However, other approaches may include one or more additional filters, e.g., for filtering or attenuating high frequency noise (e.g., > 1kHz).

The feedback comb filter 111 acts as a resonator for extracting or isolating a version of the power-line interference signal from the cardiac signal. Generally, a resonator has a circular memory (i.e., feedback) that behaves analogously to a resonance chamber, to thereby tune on all power-line interference harmonics simultaneously. A feedback comb filter is also able to automatically adapt to variations in power-line interference amplitude and phase.

$$PLI_n = \alpha(PLI_{n-D} - x_n) + x_n \qquad (1)$$

Equation (1) demonstrates one example feedback comb filter that is suitable for use in extracting a version of the power-line interference signal $PLI_n$ from the cardiac signal $x_n$. $\alpha$ is a scaling factor, which influences the accuracy and settle time of the feedback comb filter. The greater the value of $\alpha$, the greater the accuracy and settle time of the feedback comb filter. D represents a delay length. The value of $\alpha$ may be variable between 0.0 and 1.0.

The peak-frequencies of the comb filter match the fundamental PLI frequency and all harmonics when the length of the delay-line (D) exactly matches the fundamental period time of the power-line interference signal. Thus, the accuracy of the output of the feedback comb filter is enhanced when the length of the delay-line (D) exactly matches the fundamental period time of the power-line interference signal. For this reason, for improved accuracy, the length of the delay line may be fractional.

Of course, it will be appreciated that equation (1) is cast in a discrete-time format. This facilitates ease of processing (e.g., using a digital platform). However, equation (1) could be reformulated in a continuous time format, e.g., for processing a signal x(t) to produce a version of a power line interference signal PLI(t).

To be more specific, the feedback comb filter represented by equation (1) is 1st order low-pass filter (specifically a backward Euler transformed, first order lowpass filter) which is frequency scaled.

Equation (2) represents the discrete-time system function for the feedback comb filter defined in equation (1).

$$H_{res}(z) = \frac{1 - \alpha}{1 - \alpha . z^{-D}} \qquad (2)$$

For the sake of improved understanding, Figure 2 is a block diagram illustrating the steps performed to process the cardiac signal $x_n$ to produce the first version of the power-line interference signal $PLI_n$ according to the above-described feedback comb filter. Thus, Figure 2 illustrates the processes performed by the feedback comb filter 111 that operates according to equations (1) and (2).

The cardiac signal $x_n$ is summed, in a summing process 210, with a first feedback signal $FB1_n$, to produce the first version of the power-line interference signal $PLI_n$.

The first version of the power-line interference signal is delayed in a delaying step 220 by a delay length D to produce a delayed PLI signal $PLID_n$. The delay length may be defined by a coefficient $c_n$. The coefficient $c_n$ may be responsive to a predicted or calculated fundamental frequency of the power-line interference signal in the cardiac signal.

The cardiac signal $x_n$ may then be subtracted (in a subtracting step 230) from the delated PLI signal $PLID_n$, to produce a second feedback signal $FB2_n$.

**EP 4 257 033 A1**

The second feedback signal $FB2_n$ may then be multiplied, in a multiplying step 240, by a/the scaling factor $\alpha$ to produce the first feedback signal FB1 (which is then used in the summing process 210). The value of the scaling factor defines or controls the settle time of the feedback comb filter 111. The closer the value of the scaling factor to 1, the larger the settle time and the more accurate the feedback comb filter.

The feedback comb filter acts to reconstruct the periodicity in the cardiac signal $x_n$. The accuracy and speed of this reconstruction depends on the scaling factor $\alpha$. For small values of $\alpha$, the feedback comb filter needs little time to settle (e.g., <100ms). A short settling time implies that the first version of the power-line interference signal is an inaccurate representation of the true power-line interference signal in the cardiac signal. For large values of $\alpha$ the feedback comb filter needs a long time to settle (e.g. >500ms or >1s). A long settling time implies that the first version of the power-line interference signal is a more accurate representation of the true power-line interference signal in the cardiac signal.

The skilled person would be readily capable of preparing circuitry for performing a feedback comb filter, such as those previously described. The feedback comb filter may be implemented using analogue or digital circuitry. One example analogue circuitry that could be used is a bucket brigade device. Other suitable forms of analogue delay lines would be apparent.

Alternative feedback comb filters would also be readily apparent to the person skilled in the art. In particular, the described feedback comb filter is an embodiment of a more general class of suitable feedback comb filters that are based on the general property of frequency scaling.

In particular, when unity delay elements within a general system function H(z) are replaced with time-shift of M, then this results in periodicity of transfer function H(z). Matching M with the fundamental frequency of the power-line interference signal will result in notches (i.e. a feedback comb filter) at multiples of this fundamental frequency, i.e. in the harmonics of the fundamental frequency. Thus, any suitable feedback filter that makes use of a time-shift delay scaling in this way could be used as a feedback comb filter for various embodiments. Such feedback comb filters are likely to still rely upon one or more scaling factors for use in scaling any feedback signals in order to tune the settle time and/or accuracy of the filter.

Turning back to Figure 1, the proposed approach then subtracts the first version of the power-line interference signal $PLI_n$ from the cardiac signal $x_n$ to produce a filtered cardiac signal $y_n$. This subtraction takes place in a subtraction process 120.

As mentioned previously, it is recognized that a feedback comb filter may require values for one or more parameters or coefficients for appropriate and accurate operation. For instance, a feedback comb filter may require information for a delay length or length of a delay line (which represents a resonant, dominant or fundamental frequency of the power-line interference) and/or a scaling factor (which defines a tuning speed or settle time of the feedback comb filter). Generally, the greater the settle time of a feedback comb filter, the more accurate its output.

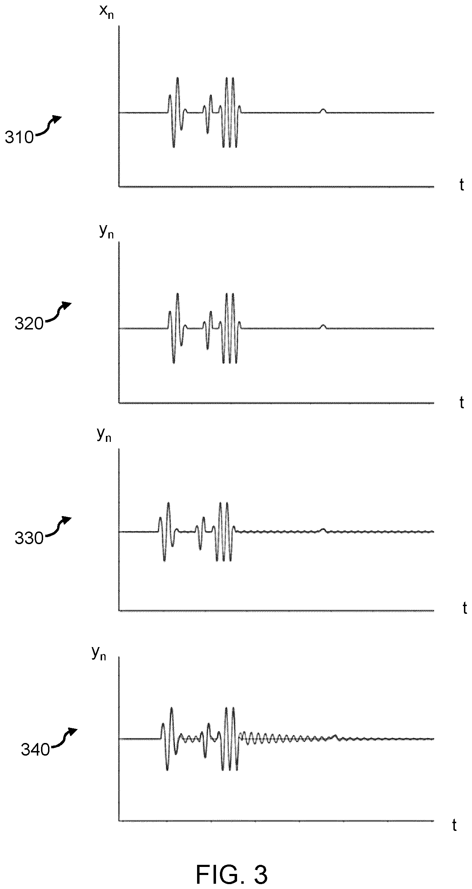

Figure 3 illustrates the effect of different values for the scaling factor $\alpha$ in the outcome of the filtered cardiac signal $y_n$, for an approach that makes use of the feedback comb filter explained with reference to equations (1) and (2), as well as Figure 2.

Each waveform illustrated in Figure 3 demonstrates a (filtered) cardiac signal $x_n$, $y_n$, against time t.

A first waveform 310 illustrates the "ground truth" cardiac signal, being a version of the cardiac signal that is free of any power-line interference whatsoever. The illustrated cardiac signal is a His-Bundle Electrogram signal, although this is used purely for the sake of example.

A second waveform 320 illustrates a first filtered cardiac signal, in which the cardiac signal $x_n$ is filtered by the feedback comb filter in which the scaling factor $\alpha$ has a value of 0.999. The first filtered cardiac signal very closely resembles the ground truth cardiac signal.

A third waveform 330 illustrates a second filtered cardiac signal, in which the cardiac signal $x_n$ is filtered by the feedback comb filter in which the scaling factor $\alpha$ has a value of 0.99. The second filtered cardiac signal closely resembles the ground truth cardiac signal, but the filter yields some "ringing effect" (i.e. noise) resulting from the frequency components of the cardiac signal which are close to the power-line interference frequencies. This has a negative effect on the morphology (i.e. amplitudes and phase) of the filtered cardiac signal. The nature of a ringing effect disturbs or distorts the cardiac signal, making it more difficult to interpret and/or identify relevant features.

A fourth waveform 340 illustrates a third filtered cardiac signal, in which the cardiac signal $x_n$ is filtered by the feedback comb filter in which the scaling factor $\alpha$ has a value of 0.9. The ringing effect is significantly more pronounced in the third filtered cardiac signal, significantly disrupting or distorting the ground-truth cardiac signal.

In some examples, the approach may use predetermined coefficients or values. For instance, it may be assumed that the resonant frequency will be the expected dominant frequency of a mains supply that powers the system producing the cardiac signal, e.g., around 50Hz or 60Hz, depending upon the jurisdiction. A fixed scaling factor could be used. For instance, experimentation has identified that a value for $\alpha$ (in equation (1)) of from 0.95 to 1 (including 0.95 but not including 1) is suitable, e.g. a value of from 0.97 to 1 (including 0.97 but not including 1), e.g. a value of from 0.99 to 1 (including 0.99 but not including 1). Thus, in some embodiments: $0.95 \le \alpha < 1$; $0.95 \le \alpha < 1$; or $0.99 \le \alpha < 1$.

6

However, in improved examples, appropriate values are determined and/or modified using a power-line interference tracker 130 to produce one or more value(s) for the feedback comb filter.

For instance, the power-line interference tracker may be configured to (continuously) estimate the dominant or fundamental frequency of the power-line interference signal in the cardiac signal. The estimated frequency may then be used to adapt the delay length for the feedback comb filter. Thus, the delay length for the feedback comb filter may be responsive to the identified fundamental frequency of the power-line interference signal.

Any suitable method for frequency estimation could be employed, such as an FFT (Fast Fourier Transform) or an STFFT (Short-Time FFT) algorithm (being a version of an FFT algorithm) could be applied.

Approaches for converted a frequency into a delay time would be readily apparent to the skilled person, e.g., by determining the reciprocal of the delay time. Another approach is to employ Lagrange interpolation to create a virtual tap on a fixed-length discrete-time delay-line.

One embodiment of the invention recognizes that although a long settle time for a feedback comb filter results in highly accurate filtering of the cardiac signal, sudden changes in the amplitude and/or phase of the power-line interference signal can take a while to propagate through. Thus, if such a change occurs, there is a relatively long period of time before the filtered cardiac signal will accurately represent the true cardiac signal. This is especially perceivable if the settle time of the feedback comb filter is large (for improved accuracy of the feedback comb filter).

One approach to overcoming this issue would be to choose a compromise value for any scaling factors of the feedback comb filter. For instance, for the feedback comb filter described with reference to equations (1) and (2), and Figure 2, a fixed scaling factor $\propto$ of 0.95 would provide a good compromise between accuracy and speed of response to changes in the power-line interference signal.

An improved approach is to dynamically modify the scaling factor and/or settle time of the feedback comb filter responsive to any changes in the power line interference signal. In particular, the settle time could be temporarily reduced if a change occurs, to quickly produce an improved cardiac signal, before being reverted to a larger settle time for improving the accuracy. This approach would significantly reduce the settle time of the feedback comb filter to a change in the power line interference signal without compromising on long-term accuracy.

Thus, in some examples, the approach is configured such that the power-line interference tracker 130 is configured to process the cardiac signal to detect changes in the peak amplitude and/or phase of the power-line interference signal in the cardiac signal; and responsive to a detected change in the peak amplitude and/or phase, modify the scaling factor for the feedback comb filter to reduce a settle time of the feedback comb filter.

Effectively, the power-line interference tracker may detect events in the cardiac signal representing changes in the power-line interference signal.

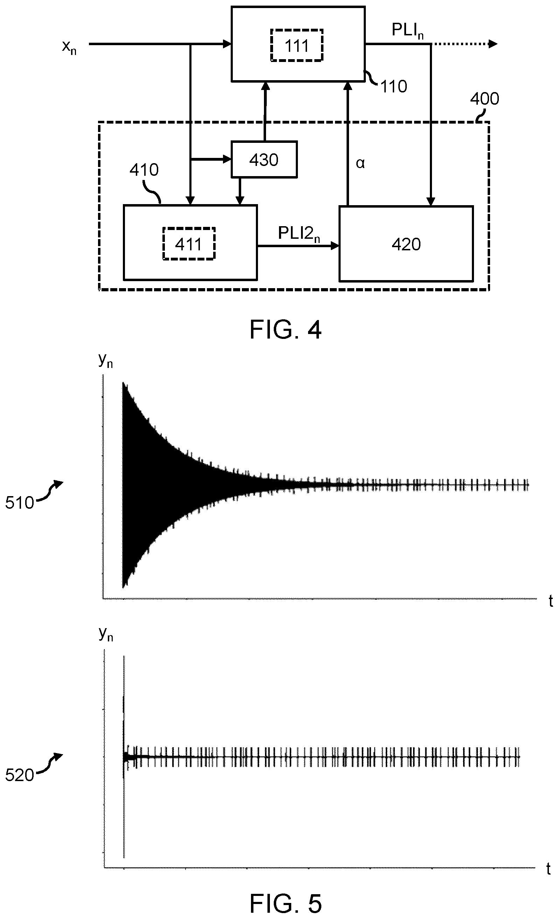

One approach for detecting changes in the peak amplitude and/or phase of the power-line interference signal in the cardiac signal is illustrated by Figure 4. Figure 4 illustrates a power-line interference tracker 400 for use in an embodiment.

The power-line interference tracking system 400 is configured to process the cardiac signal $x_n$ using only a second set 410 of one or more linear filters, including at least a second feedback comb filter 411, to extract or isolate a second version of the power-line interference signal $PLI2_n$ carried by the cardiac signal $x_n$. A settle time of the second feedback comb filter is less than the settle time of the feedback comb filter (in the first set).

The second feedback comb filter may, for instance, be the same type of feedback comb filter (e.g., be represented by a same transfer function) but with a different scaling factor.

The power-line interference signal may be configured to monitor a difference between the first version of the power-line interference signal $PLI_n$ (produced by the first set of one or more linear filters) and the second version of the power-line interference signal $PLI2_n$. This process can be performed by a change monitoring unit 420.

The difference may, for instance, be a maximum difference between the first and second versions within a predetermined preceding time period (e.g., a preceding time period of between 0.015 and 30 seconds, more preferably between 0.015 and 10 seconds, more preferably between 0.015 and 10 seconds, more preferably between 0.015 and 1 second, more preferably between 0.015 and 0.5 seconds.

In another example, the difference may be an average difference between the first and second versions within a predetermined preceding time period (e.g., a preceding time period of between 0.015 and 30 seconds, more preferably between 0.015 and 10 seconds, more preferably between 0.015 and 10 seconds, more preferably between 0.015 and 1 second, more preferably between 0.015 and 0.5 seconds.

Responsive to the difference exceeding a first predetermined difference threshold, determining that there is change in the peak amplitude and/or phase of the power-line interference signal in the cardiac signal.

If the difference is a maximum difference between the first and second versions within a predetermined preceding time period, then the first predetermined difference threshold may, for instance, be a value equal to a predetermined percentage of the maximum or peak value of the first version of the power-line interference signal or the second version of the power-line interference signal $PLI2_n$, e.g., within the predetermined preceding time period.

For instance, the first predetermined difference may be a value between 5% and 25% (e.g., 5%, 10%, 15%, 20% or 25%) of the maximum or peak value of the first version of the power-line interference signal within the predetermined preceding time period.

For instance, the first predetermined difference may be a value between 5% and 25% (e.g., 5%, 10%, 15%, 20% or 25%) of the maximum or peak value of the second version of the power-line interference signal within the predetermined preceding time period.

In some examples, the first predetermined difference may be the lesser of a value between 5% and 25% (e.g., 5%, 10%, 15%, 20% or 25%) of the maximum or peak value of the first version of the power-line interference signal within a previous third period of time and a value between 5% and 25% (e.g., 5%, 10%, 15%, 20% or 25%) of the maximum or peak value of the second version of the power-line interference signal within the predetermined preceding time period.

If the difference is a maximum difference between the first and second versions within a predetermined preceding time period, then the first predetermined difference threshold may, for instance, be a value equal to a predetermined percentage of the average value of the first version of the power-line interference signal or the second version of the power-line interference signal $PLI2_n$, e.g., within the predetermined preceding time period.

For instance, the first predetermined difference may be a value between 5% and 25% (e.g., 5%, 10%, 15%, 20% or 25%) of the average of the first version of the power-line interference signal within the predetermined preceding time period.

For instance, the first predetermined difference may be a value between 5% and 25% (e.g., 5%, 10%, 15%, 20% or 25%) of the average value of the second version of the power-line interference signal within the predetermined preceding time period.

In some examples, the first predetermined difference may be the lesser of a value between 5% and 25% (e.g., 5%, 10%, 15%, 20% or 25%) of the average value of the first version of the power-line interference signal within a previous third period of time and a value between 5% and 25% (e.g., 5%, 10%, 15%, 20% or 25%) of the average value of the second version of the power-line interference signal within the predetermined preceding time period.

As previously mentioned, the settle time (e.g., the scaling factor) of the feedback comb filter (in the first set of one or more filers) is modified responsive to a detected change in the peak amplitude and/or phase. This can be performed by the change monitoring unit 420, e.g., controlling or defining the value of the scaling factor $\alpha$ used by the feedback comb filter 111.

In particular examples, modifying the settle time for the feedback comb filter comprises decreasing the settle time of the feedback comb filter when there is (i.e. responsive to) a detected change in the peak amplitude and/or phase. This may occur, for instance, if the difference between the first version of the power-line interference signal $PLI_n$ (produced by the first set 110 of one or more linear filters) and the second version of the power-line interference signal $PLI2_n$ exceeds a first predetermined difference threshold.

Decreasing the settle time may be performed by switching a scaling factor for the feedback comb filter from a first value to a second, different value. The second value may be less than the first value, e.g., for the feedback comb filter described with reference to equations (1), (2) and Figure 2. Other suitable adaptations for other forms of feedback comb filter for appropriately modifying the settle time will be apparent to the skilled person.

For at least the feedback comb filter described with reference to equations (1), (2) and Figure 2, the first value may be a value between 0.97 and 1 (including 0.97 but excluding 1). Similarly, for at least the feedback comb filter described with reference to equations (1), (2) and Figure 2, the second value may be value between 0.5 and 0.97, more preferably between 0.5 and 0.9, more preferably between 0.5 and 0.7. In one working example, the first value may be equal to 0.999 and the second value may be equal to 0.6, which has been shown to provide a good compromise between

The settle time may be increased when the effect of the detected change has passed. Increasing the settle time may be performed, for instance, by increasing the scaling factor $\alpha$. For instance, increasing the settle time may be performed by reverting the scaling factor for the feedback comb filter from the second value to the first value.

In one example, this can be performed by simply increasing the settle time after a predetermined time period has lapsed, e.g., a time period of no less than 50ms, e.g., no less than 100ms, e.g., no less than 500ms, e.g., no less than 1 second, e.g., no less than 5 seconds, e.g., no less than 10 seconds.

The settle time $\tau_c$ for a feedback comb filter is dependent upon the value of $\alpha$ and the fundamental PLI frequency. The fundamental PLI period $T_{PLI}$ is the reciprocal of the fundamental PLI frequency. The settle time $\tau_c$ can be defined as follows:

$$\tau_c = T_{PLI} \frac{\alpha}{1 - \alpha} \qquad (3)$$

The time period after which the settle time is increased should be larger than the expected settle time $\tau_c$ calculated used equation (3). The value for $T_{PLI}$ should be set to be equal to an expected (e.g., expected lowest potential) fundamental PLI period. For instance, the value of $T_{PLI}$ may be set to 20 (representing a fundamental PLI frequency of 50Hz).

As one example, if the scaling factor is reduced to 0.6 when modifying the settle time, the scaling factor should be increased no less than 30ms since reducing the scaling factor to 0.6.

However, in a preferred example, the scaling factor for the feedback comb filter may be modified to increase the settle time of the feedback comb filter responsive to a/the monitored difference between the first version of the power-line interference signal and the second version of the power-line interference signal falling below a second predetermined difference threshold.

The second predetermined difference threshold may be the same as the first predetermined difference threshold.

However, in preferred examples, the second predetermined difference threshold is less than the first predetermined difference threshold, e.g., a value no greater than 10% of the first predetermined difference threshold, e.g., no greater than 5% of the first predetermined difference threshold. This preferred example would reduce a chance of increasing the settle time too quickly, e.g., before the benefits of a quicker settle time have been fully realized.

Figure 5 illustrates the effect of performing a switching of the settle time and/or scaling factor, such as the approach previously described.

A first waveform 510 illustrates the filtered cardiac signal $y_n$ if the settle time is fixed at a relatively long settle time, e.g., if the scaling factor for the feedback comb filter (described with reference to Figure 2 and equations (1) and (2)) is fixed at 0.999.

A second waveform 520 illustrates the filtered cardiac signal $y_n$ if the settle time is switched between a long settle time and a short settle time responsive to detection of change in the peak amplitude and/or phase of the power-line interference signal. In this scenario, the settle time is reverted to the long settle time once the feedback comb filter has settled. For instance, if the scaling factor for the feedback comb filter (described with reference to Figure 2 and equations (1) and (2)) is switched from 0.999 to 0.6 responsive to detecting such a change.

Both the first and second waveforms are plotted against time t, the scale of the time axis being equivalent.

As can be clearly seen from Figure 5, the response time of the filtered cardiac signal is significantly quicker when switching of the settle time occurs. As previously mentioned, the settle time may again be increased for the feedback comb filter when the effect of the change in the peak amplitude and/or phase of the power-line interference signal has been accounted for by the filtered cardiac signal, e.g., after a predetermined period of time has elapsed or the difference between two versions of the power-line interference signal (as previously described) is below some predetermined threshold.

Figure 4 further illustrates some other optional elements for the power-line interference tracker 400.

The power-line interference tracker 400 may comprise a frequency determination unit 430 configured to determine or estimate the dominant or fundamental frequency of the power-line interference signal in the cardiac signal $x_n$. Any suitable method for frequency estimation could be employed, such as an FFT (Fast Fourier Transform) or an STFFT (Short-Time FFT) algorithm (being a version of an FFT algorithm) could be applied.

As previously explained, the power-line interference tracker may be configured to adapt the delay length for the feedback comb filter 111 of the first set of one or more linear filters. In particular, the delay length s preferably configured to match the period of the power-line interference signal (i.e., the reciprocal of the dominant frequency).

The power-line interference tracker may be configured to adapt the delay length for the second feedback comb filter 411 of the second set 410 of one or more linear filters. In particular, the delay length may be configured to match the period of the power-line interference signal (i.e., the reciprocal of the dominant frequency). This is illustrated in Figure 4.

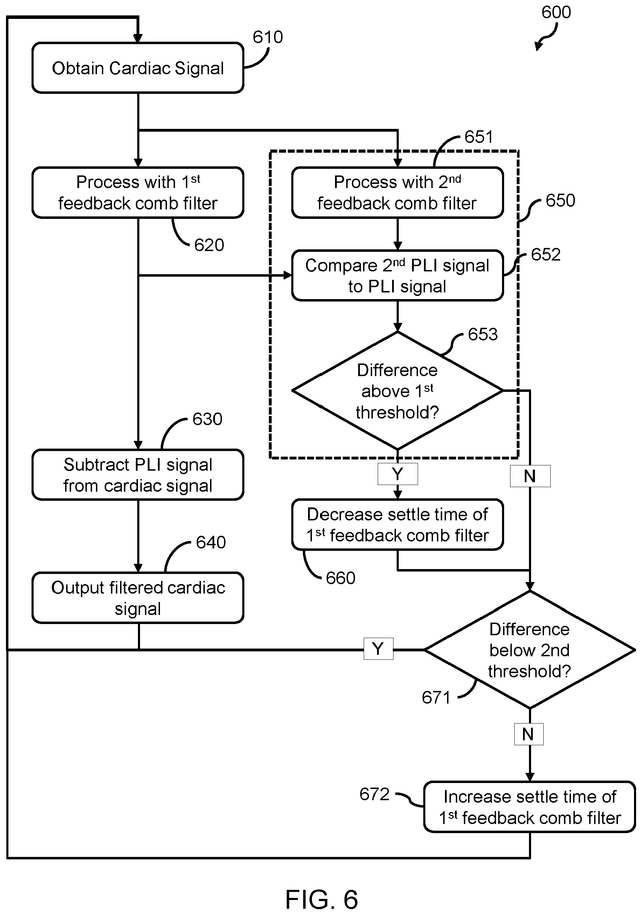

For the sake of completeness, Figure 6 illustrates a method 600 according to an embodiment.

The method 600 comprises a step 610 of obtaining a cardiac signal. The cardiac signal may be obtained at a user interface. The cardiac signal is representative of electrical activity of a portion of a subject's heart.

The method 600 also comprises a step 620 of processing the cardiac signal using only a first set of one or more linear filters, including at least a feedback comb filter, to extract or isolate a first version of a power-line interference signal in the cardiac signal.

The method 600 also comprises a step 630 of subtracting the reconstructed first version of the power-line interference signal from the cardiac signal to produce a filtered cardiac signal,

The method 600 may comprise a step 640 of outputting the filtered cardiac signal. The method 600 may be iteratively repeated.

In some examples, the method also comprises a process 650 of processing the cardiac signal to detect changes in the peak amplitude and/or phase of the power-line interference signal in the cardiac signal.

Responsive to detecting a change in the peak amplitude and/or phase, the method 600 may perform a step 660 of modifying the scaling factor for the feedback comb filter to reduce a settle time of the feedback comb filter. Otherwise, step 660 may be skipped.

Process 650 can be performed by performing a step 651 of processing the cardiac signal using only a second set of one or more linear filters, including at least a second feedback comb filter, to extract or isolate a second version of the power-line interference signal carried by the cardiac signal, wherein a settle time of the second feedback comb filter is less than the settle time of the feedback comb filter.

Process 650 may also comprise a step 652 of monitoring a difference between the first version of the power-line interference signal and the second version of the power-line interference signal.

Process 653 may also comprise a step 653 of determining whether the difference exceeds a first predetermined difference threshold to determining whether or not there is a change in the peak amplitude and/or phase of the power-line interference signal in the cardiac signal.

Optionally, the method 600 is further configured to determine whether the difference (determined in step 652) is below a second predetermined threshold in a step 671. If a positive determination is made in step 671, the method 600 performs a step 672 of modifying the scaling factor for the feedback comb filter to increase a settle time of the feedback comb filter. Otherwise, step 672 may be skipped.

As before, the method 600 may be iteratively repeated.

The skilled person would be readily capable of developing a processing system for carrying out any herein described method. Thus, each step of the flow chart may represent a different action performed by a processing system, and may be performed by a respective module of the processing system.

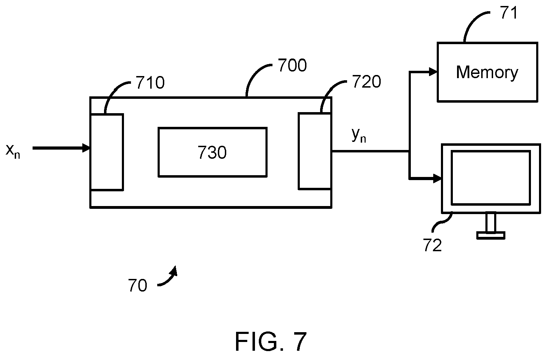

Figure 7 illustrates an exemplary processing system 700 for improved understanding. The processing system 700 is for filtering a cardiac signal and is illustrated in the context of a processing apparatus 70, which is itself an embodiment of the invention.

The processing system 700 comprises an input interface 710 configured to obtain a cardiac signal $x_n$ representative of electrical activity of a portion of a subject's heart.

The processing system 700 also comprises a processing unit 720 configured to process the cardiac signal using only a first set of one or more linear filters, including at least a feedback comb filter, to extract or isolate a first version of a power-line interference signal in the cardiac signal; and subtract the first version of the power-line interference signal from the cardiac signal to produce a filtered cardiac signal $y_n$.

The processing system 700 may comprise an output interface configured to output the filtered cardiac signal $y_n$.

The processing unit 720 may be configured to perform any other suitable action or process for generating the filtered cardiac signal, e.g., monitoring for parameters of and/or changes in the power-line interference signal and appropriately modifying, controlling or defining one or more coefficients or values for the feedback comb filter.

The filtered cardiac signal may be further processed (e.g. by a further processing unit), stored (e.g., in a memory or recording system) and/or presented to an individual, e.g., in the form of a visual representation of the filtered cardiac signal.

Thus, the processing system may be configured to store the filtered cardiac signal in a memory or recording system 71 of the apparatus 70, which forms an optional feature of the apparatus 70.

Similarly, the processing system may be configured to control a user interface 72 or display to provide a visual representation of the filtered cardiac signal. The user interface 72 may form part of the apparatus 70.

The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

Thus, the processing system may comprise or be a field-programmable gate array or an application-specific integrated circuit.

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method. In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa. Any reference signs in the claims should not be construed as limiting the scope.

**Claims**

1. A processing system (100, 700) for filtering a cardiac signal ($x_n$) representative of electrical activity of a portion of a subject's heart, the processing system being configured to:

   obtain (610), at an input interface (710);
   process (620) the cardiac signal using only a first set (110) of one or more linear filters, including at least a feedback comb filter (111), to extract or isolate a first version of a power-line interference signal ($PLI_n$) in the cardiac signal,
   subtract (630) the first version of the power-line interference signal from the cardiac signal to produce a filtered cardiac signal ($y_n$); and
   optionally, output (640), at an output interface (720), the filtered cardiac signal.

2. The processing system of claim 1, wherein the processing system is further configured to:

   process (650) the cardiac signal to detect changes in the peak amplitude and/or phase of the power-line interference signal in the cardiac signal; and
   responsive to a detected change in the peak amplitude and/or phase, modify (660) the scaling factor for the feedback comb filter to reduce a settle time of the feedback comb filter.

3. The processing system of claim 2, wherein the processing system is configured to detect changes in the peak amplitude and/or phase of the power-line interference signal in the cardiac signal by:

   processing (651) the cardiac signal using only a second set (410) of one or more linear filters, including at least a second feedback comb filter (411), to extract or isolate a second version of the power-line interference signal ($PLI2_n$) carried by the cardiac signal, wherein a settle time of the second feedback comb filter is less than the settle time of the feedback comb filter;
   monitoring (652) a difference between the first version of the power-line interference signal and the second version of the power-line interference signal; and
   responsive to a difference exceeding a first predetermined difference threshold, determining that there is change in the peak amplitude and/or phase of the power-line interference signal in the cardiac signal.

4. The processing system of claim 3, wherein the processing system is further configured to, responsive to the monitored difference between the first version of the power-line interference signal and the second version of the power-line interference signal falling below a second predetermined difference threshold, modify (672) the scaling factor for the feedback comb filter to increase the settle time of the feedback comb filter.

5. The processing system of claim 4, wherein:

   the processing system is configured to modify the scaling factor for the feedback comb filter to decrease the settle time of the feedback comb filter by changing the scaling factor from a first value to a second, different value; and

the processing system is configured to modify the scaling factor for the feedback comb filter to increase the settle time of the feedback comb filter by changing the scaling factor from the second value to the first value.

6. The processing system of any of claims 1 to 5, wherein the feedback comb filter is configured to generate the first version of the power-line interference signal by:

   summing (210) the cardiac signal with a first feedback signal to produce the first version of the power-line interference signal;
   delaying (220) the first version of the power-line interference signal by a delay length to produce a delayed PLI signal;
   subtracting (230) the cardiac signal from the delayed PLI signal to produce a second feedback signal; and
   multiplying (240) the second feedback signal by a scaling factor to produce the first feedback signal.

7. The processing system of any of claims 1 to 6, wherein the processing system is further configured to process the cardiac signal to identify a fundamental frequency of a power-line interference signal in the cardiac signal, wherein a delay length for the feedback comb filter is responsive to the identified fundamental frequency of the power-line interference signal.

8. The processing system of claim 7, wherein the processing system is configured to process the cardiac signal to identify a resonant frequency of the power-line interference signal in the cardiac signal by performing a discrete Fourier-based transform on the cardiac signal.

9. The processing system of claim 8, wherein the discrete Fourier-based transform comprises a fast Fourier transform or a short-time fast Fourier transform.

10. The processing system of any of claims 1 to 9, wherein the cardiac signal is a high right atrial electrogram signal, a his-bundle electrogram signal or an electrocardiogram signal.

11. The processing system of any of claims 1 to 10, wherein the cardiac signal is produced by an intravenous monitoring device such as a catheter.

12. The processing system of any of claims 1 to 11, wherein the processing system is further configured to store the filtered cardiac signal in a memory or recording system.

13. A computer-implemented method (600) for filtering a cardiac signal $(x_n)$ representative of electrical activity of a portion of a subject's heart, the computer-implemented method comprising:

   obtaining (610) the cardiac signal;
   processing (620) the cardiac signal using only a first set (110) of one or more linear filters, including at least a feedback comb filter (111), to extract or isolate a first version of a power-line interference signal $(PLI_n)$ in the cardiac signal;
   subtracting (630) the first version of the power-line interference signal from the cardiac signal to produce a filtered cardiac signal $(y_n)$; and
   optionally, outputting (640) the filtered cardiac signal.

14. The computer-implemented method of claim 13, further comprising:

   processing (650) the cardiac signal to detect changes in the peak amplitude and/or phase of the power-line interference signal in the cardiac signal; and
   responsive to a detected change in the peak amplitude and/or phase, modifying (660) the scaling factor for the feedback comb filter to reduce a settle time of the feedback comb filter.

15. A computer program product comprising computer program code means which, when executed on a computing device having a processing system, cause the processing system to perform all of the steps of the method according to any of claims 13 or 14.

FIG. 1

FIG. 2

FIG. 3

FIG. 4

FIG. 5

600

610
Obtain Cardiac Signal

Process with 1st
feedback comb filter
620

Process with 2nd
feedback comb filter
651

650

Compare 2nd PLI signal
to PLI signal
652

630
Subtract PLI signal
from cardiac signal

Difference
above 1st
threshold?
653

Y

N

640
Output filtered cardiac
signal

Decrease settle time of
1st feedback comb filter
660

Y

Difference
below 2nd
threshold?

671

N

672
Increase settle time of
1st feedback comb filter

FIG. 6

FIG. 7

**EUROPEAN SEARCH REPORT**

Europäisches Patentamt
European Patent Office
Office européen des brevets

Application Number

EP 22 16 6556

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | YOSUKE SUGIURA1) ET AL: "A Comb Filter with Adaptive Notch Gain and Bandwidth", IEICE TRANSACTIONS ON FUNDAMENTALS OF ELECTRONICS,COMMUNICATIONS AND COMPUTER SCIENCES, ENGINEERING SCIENCES SOCIETY, TOKYO, JP, vol. E96A, no. 4, 1 April 2013 (2013-04-01), pages 790-795, XP001584217, ISSN: 0916-8508, DOI: HTTP://DX.DOI.ORG/10.1587/TRANSFUN.E96.A.790 [retrieved on 2013-04-01] | 1,2,6-15 | INV. A61B5/00 |
| A | * abstract * * 1. Introduction p. 790 – 791. 2. Comb filter with adaptive notch gain, p. 791 – 792. * | 3-5 | |
| Y | MENGARELLI ALESSANDRO ET AL: "A MATLAB-based graphical user interface for the identification of muscular activations from surface electromyography signals", 2016 38TH ANNUAL INTERNATIONAL CONFERENCE OF THE IEEE ENGINEERING IN MEDICINE AND BIOLOGY SOCIETY (EMBC), IEEE, 16 August 2016 (2016-08-16), pages 3646-3649, XP032979960, DOI: 10.1109/EMBC.2016.7591518 [retrieved on 2016-10-13] | 1,2,6-15 | TECHNICAL FIELDS SEARCHED (IPC) A61B |
| A | * III. A. Further features, p. 3648 * | 3-5 | |

-/--

The present search report has been drawn up for all claims

| Place of search | Date of completion of the search | Examiner |
|---|---|---|
| Berlin | 24 August 2022 | Trachterna, Morten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

Application Number

EP 22 16 6556

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JANG Y K ET AL: "ADAPTIVE IIR COMB FILTER FOR HARMONIC SIGNAL CANCELLATION", INTERNATIONAL JOURNAL OF ELECTRONICS, TAYLOR AND FRANCIS.LTD. LONDON, GB, vol. 75, no. 2, 1 August 1993 (1993-08-01), pages 241-250, XP000385418, ISSN: 0020-7217 | 1,2,6-15 | |
| A | * abstract * <br> * 2. IIR filter model structure for harmonic signal cancellation p. 242 - 243 * | 3-5 | |
| Y | Anonymous: "Feedback Comb Filters", , 28 July 2007 (2007-07-28), pages 1-2, XP055954568, Retrieved from the Internet: URL:https://web.archive.org/web/2007072806 4835/https://ccrma.stanford.edu/~jos/pasp0 4/Feedback_Comb_Filters.html [retrieved on 2022-08-24] | 1,2,6-15 | |
| A | * the whole document * | 3-5 | TECHNICAL FIELDS SEARCHED (IPC) |
| A | WU Y ET AL: "Filtering electrocardiographic signals using an unbiased and normalized adaptive noise reduction system", MEDICAL ENGINEERING & PHYSICS, BUTTERWORTH-HEINEMANN, GB, vol. 31, no. 1, 1 January 2009 (2009-01-01), pages 17-26, XP025841995, ISSN: 1350-4533, DOI: 10.1016/J.MEDENGPHY.2008.03.004 [retrieved on 2008-05-09] * abstract * * 2.2 Comb filtering with an IIR comb filter p. 18 - 19 * | 1-15 | |

The present search report has been drawn up for all claims

| Place of search | Date of completion of the search | Examiner |
|---|---|---|
| Berlin | 24 August 2022 | Trachterna, Morten |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

page 2 of 2